(19) Europäisches Patentamt
European Patent Office
Office européen des brevets

(11) **EP 2 597 969 B1**

(12) **EUROPEAN PATENT SPECIFICATION**

(45) Date of publication and mention
of the grant of the patent:
**14.10.2020 Bulletin 2020/42**

(21) Application number: **11741728.7**

(22) Date of filing: **29.07.2011**

(51) Int Cl.:
*A23L 2/52* (2006.01)        *A61K 36/752* (2006.01)
*A61Q 19/00* (2006.01)      *C11D 3/382* (2006.01)
*A23K 10/37* (2016.01)      *A23L 29/206* (2016.01)
*A23L 19/00* (2016.01)      *A23L 33/22* (2016.01)
*A61K 8/9789* (2017.01)

(86) International application number:
**PCT/US2011/045975**

(87) International publication number:
**WO 2012/016190 (02.02.2012 Gazette 2012/05)**

(54) **PROCESS FOR OBTAINING CITRUS FIBER FROM CITRUS PULP**

VERFAHREN ZUR GEWINNUNG VON ZITRUSFASERN AUS ZITRUSPULPE

PROCÉDÉ POUR OBTENIR UNE FIBRE D'AGRUME À PARTIR DE PULPE D'AGRUME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.08.2010 EP 10008317
30.07.2010 US 369204 P**

(43) Date of publication of application:
**05.06.2013 Bulletin 2013/23**

(60) Divisional application:
**19150727.6 / 3 542 642**

(73) Proprietor: **Cargill, Incorporated
Wayzata, Minnesota 55413 (US)**

(72) Inventors:
• **GUSEK, Todd Walter
Crystal
MN 55422 (US)**
• **MAZOYER, Jacques Andre Christian
F-50500 Carentan (FR)**
• **REEDER, David Hiram
Chanhassen
MN 55317 (US)**
• **WALLECAN, Joel Rene Pierre
B-1800 Vilvoorde (BE)**

(74) Representative: **Morariu Radu, Mihai Dorin et al
Cargill R&D Centre Europe BVBA
Bedrijvenlaan 9
2800 Mechelen (BE)**

(56) References cited:
**WO-A1-2006/033697        WO-A1-2009/075851
WO-A1-2010/060778        US-B1- 6 183 806
US-B2- 7 094 317**

Remarks:
The file contains technical information submitted after the application was filed and not included in this specification

**Description**

TECHNICAL FIELD

The present invention is directed to a process for obtaining citrus fiber from citrus pulp. The resulting dried citrus fiber is useful as a food additive in food products, feed products and beverages. The citrus fiber is also useful in personal care, pharmaceutical or detergent products.

BACKGROUND OF THE INVENTION

The prior art describes methods for extracting citrus fiber from citrus pulp.

For example, U.S. Patent No 7,094,317 (Fiberstar, Inc.) describes a process for refining cellulosic material from organic fiber plant mass (such as citrus fruit). The process discloses a first step of soaking the organic fiber plant mass in an aqueous solution, draining the organic fiber plant mass and allowing it to sit for sufficiently long time to enable cells in the organic fiber plant mass to open cells and expand the organic fiber plant mass. The soaking step requires at least 4 hours and is reported to be critical to get the materials to fully soften. The soaked raw material is then refined under high shear and dried.

W.O. Patent Application No 94/27451 (The Procter & Gamble Company) describes a process for producing a citrus pulp fiber, wherein first an aqueous slurry of citrus pulp is prepared which is then heated to a temperature of 70°C to 180°C for at least 2 minutes. The slurry is then subjected to a high shear treatment.

W.O. Patent Application No 2006/033697 (Cargill, Inc.) describes a process of extracting citrus fiber from citrus vesicles. The process includes washing citrus vesicles with water, contacting the water washed vesicles with an organic solvent to obtain organic solvent washed vesicles, desolventizing the organic solvent washed vesicles and recovering dried citrus fiber therefrom.

U.S. Patent No 7,094,317 discloses treating citrus pulp using pressure homogenization and then drying the homogenized material in a dehydrator.

While the prior art reports that citrus fiber with useful properties is obtained, there remains a need to further improve the characteristics of citrus fiber.

Hence, it is an object of the present invention to develop a process for obtaining citrus fiber from citrus pulp having improved properties versus the citrus fiber of the prior art. It is further an object of the present invention to obtain a citrus fiber which has good hydration ability and viscosifying properties.

SUMMARY OF THE INVENTION

The present invention, according to one aspect, is directed to a process for obtaining citrus fiber from citrus pulp. In one embodiment, citrus pulp is treated to obtain homogenized citrus pulp. The process further comprises a step of washing the homogenized citrus pulp with an organic solvent to obtain organic solvent washed citrus pulp. The organic solvent washed citrus pulp is desolventized and dried, and citrus fiber is recovered.

In another aspect the present invention is directed to a citrus fiber having a c* close packing concentration value of less than 3.8.

In a preferred embodiment, the citrus fiber has a viscosity of at least 1000 mPa.s, wherein said citrus fiber is dispersed in standardized water at a mixing speed of from 800 rpm to 1000 rpm, preferably 900 rpm, to a 3 w/w% citrus fiber/standardized water solution, and wherein said viscosity is measured at a shear rate of 5 $s^{-1}$ at 20°C. In another preferred embodiment, the citrus fiber has a CIELAB L* value of at least 90.

Further disclosed is a blend of citrus fiber of the present invention and plant-derived (e.g. derived from cereals) fiber, citrus fiber obtained from citrus peel or citrus rag (segment membranes, core) and combinations thereof.

Further disclosed is a food product, a feed product, a beverage, a personal care product, pharmaceutical product or a detergent product comprising the citrus fiber according to the present invention.

Further disclosed is the use of the citrus fiber as a texturiser or viscosifier in food products, feed products, beverages, personal care product, pharmaceutical product or detergent product.

BRIEF DESCRIPTION OF THE DRAWINGS

FIG. 1 is a schematic illustration of a process in accordance with a preferred embodiment of the present invention.

Figs. 2a and 2b are an illustration in accordance with a test method used in the present application.

DETAILED DESCRIPTION OF THE INVENTION

In one aspect, the present invention is directed to a process for obtaining citrus fiber from citrus pulp.

The term "citrus pulp," as used herein, refers to the pectinaceous and cellulosic material contained in the inner, juice-containing portion of citrus fruit. It is a co-product of the orange juice industry, resulting from the mechanical extraction of the juice. Citrus pulp is sometimes also referred to as coarse pulp, floaters, citrus cells, floating pulp, juice sacs, citrus vesicles or pulp. Citrus pulp typically has a water content of at least about 80 wt% and usually from about 90 to about 98 wt%.

The term "citrus fiber," as used herein, refers to a fibrous pecto-cellulosic component obtained from citrus pulp.

The process according to the present invention may be used for obtaining citrus fiber from citrus pulp from a wide variety of citrus fruit, non-limiting examples of which include oranges, tangerines, limes, lemons, and grapefruit. In one preferred embodiment, citrus fiber is obtained from orange pulp.

In the process according to the present invention, citrus pulp is treated to obtain homogenized citrus pulp. Optionally, the citrus pulp may be washed with water prior to homogenization. Sometimes, citrus pulp may be provided in a frozen or dried state which requires thawing or rehydration prior to homogenization. Preferably, the citrus pulp is adjusted with water to a dry matter content of 5 wt% or less. While not intending to be bound to any theory, it is believed that the homogenization treatment causes disruption and disintegration of whole pulp cells and cell fragments. Homogenization can be effected by a number of possible methods including, but not limited thereto, high shear treatment, pressure homogenization, colloidal milling, intensive blending, extrusion, ultrasonic treatment, and combinations thereof. Preferably, the power input (power per unit volume) for effecting homogenization is at least 1000 kW per $cm^3$ of citrus pulp.

In the present invention, the homogenization treatment is a pressure homogenization treatment. Pressure homogenizers typically comprise a reciprocating plunger or piston-type pump together with a homogenizing valve assembly affixed to the discharge end of the homogenizer. Suitable high pressure homogenizers include high pressure homogenizers manufactured by GEA Niro Soavi, of Parma (Italy), such as the NS Series, or the homogenizers of the Gaulin and Rannie series manufactured by APV Corporation of Everett, Massachusetts (US).

During the pressure homogenization, the citrus pulp is subjected to high shear rates as the result of cavitation and turbulence effects. These effects are created by the citrus pulp entering the homogenizing valve assembly from the pump section of the homogenizer at a high pressure (and low velocity). Suitable pressures for the process of the present invention are from 50 bar to 1000 bar.

Depending on the particular pressure selected for the pressure homogenization, and the flow rate of the citrus pulp through the homogenizer, the citrus pulp may be homogenized by one pass through the homogenizer. However, more than one pass of the citrus pulp may be required.

In one embodiment, the citrus pulp is homogenized by a single pass through the homogenizer. In a single pass homogenization, the pressure used is preferably from 300 bar to 1000 bar, more preferably from 400 bar to 800 bar, even more preferably from 500 bar to 750 bar.

In another preferred embodiment, the citrus pulp is homogenized by multiple passes through the homogenizer, preferably at least 2 passes, more preferably at least 3 passes through the homogenizer. In a multipass homogenization, the pressure used is typically lower compared to a single-pass homogenization and preferably from 100 bar to 600 bar, more preferably from 200 bar to 500 bar, even more preferably from 300 bar to 400 bar.

Optionally, the citrus pulp may be subjected to a heat treatment prior to homogenization. Preferably, the temperature used in the heat treatment can vary from 50°C to 140°C for a period of from 1 second to 3 minutes. The heat treatment may be used for pasteurization of the citrus pulp. For pasteurization, the heat treatment preferably employs a temperature of from 65°C to 140°C, preferably from 80°C to 100°C for a period of from 2 seconds to 60 seconds, preferably from 20 seconds to 45 seconds. Pasteurization is preferred to inactivate pectinesterases for preventing cloud loss and to inactivate spoilage micro-organisms for enhancing storage stability.

The homogenized citrus pulp is then contacted with an organic solvent. In one aspect, the organic solvent extracts water, flavors, odors, colors and the like from the citrus pulp. The solvent should preferably be polar and water-miscible to better facilitate removal of the desired components. Available solvents may include lower alcohols such as methanol, ethanol, propanol, isopropanol, or butanol. Preferred solvents are ethanol, isopropanol, and combinations thereof. The solvent may be provided in aqueous solution. The concentration of solvent in the solvent solution most often ranges from about 70 wt% to about 100 wt%. In one embodiment, a 75 wt% aqueous ethanol solution is used as solvent. In a preferred embodiment, a 90 wt% aqueous ethanol solution is used as solvent. In general, solvents will remove water-soluble components at lower concentrations and oil- soluble components at higher concentrations. Optionally, a more non-polar co- solvent may be added to the aqueous alcohol to improve the recovery of oil-soluble components in the citrus pulp. Examples of such non-polar solvents include ethyl acetate, methyl ethyl ketone, acetone, hexane, methyl isobutyl ketone and toluene. The more non-polar solvents may be added at up to 20% of the solvent mixture. Many solvents, such as ethanol, have a lower heat of vaporization than that of water, and therefore require less energy to volatilize than would be needed to volatilize an equivalent mass of water. The solvent preferably is removed and reclaimed for reuse.

Preferably, the citrus pulp is contacted with organic solvent at a solids-to-solvent weight ratio of at least about 0.25:1, preferably at least about 0.5:1, and often at least about 0.75:1, from about 1 :1 to about 5:1, or from about 1.5:1 to about 3:1, based on the wet weight of the solids. In one embodiment, the solids-to-solvent ratio is about 2:1.

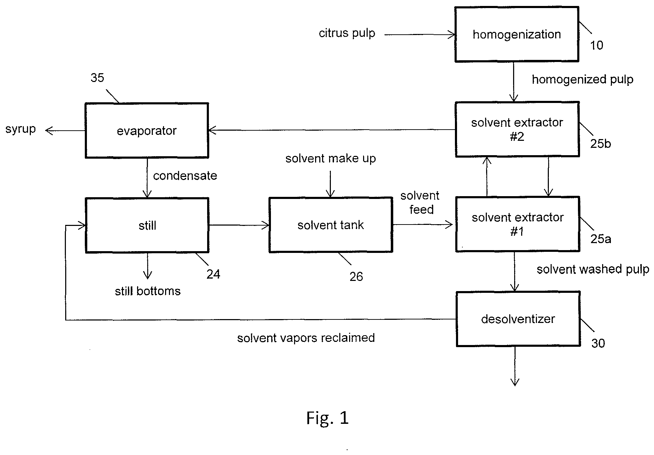

Extraction can be accomplished using a single stage but preferably is performed using multistage extraction, e.g., a two-, three-, or four-staged extraction process, and preferably using countercurrent extraction. There is no particular upper limit contemplated on the number of extraction stages that may be used. Fig. 1 schematically illustrates a preferred embodiment in which a two-stage countercurrent extraction process employs first and second solvent extractors **25a** and **25b**, respectively.

After homogenization **10**, homogenized citrus pulp is fed into the second extractor **25b**. An aqueous ethanol solvent is fed from a solvent tank **26** into the first solvent extractor **25a**. Spent solvent from the first solvent extractor **25a** is fed into the second solvent extractor **25b**, while the extracted citrus pulp from the second solvent extractor **25b** are fed into the first solvent extractor **25a**. Spent solvent from the second solvent extractor **25b** may be fed into an evaporator **35** (optional) to separate solids (e.g., sugars, acids, colors, flavors, citrus oils, etc.) from the spent solvent, which can be condensed and returned to a still **24**. Still bottoms (predominately water) are separated and removed.

After each extraction stage, liquid is preferably further removed. One suitable device is a decanter centrifuge. Alternatively, a sieve, a belt filter press or other device suitable for removing liquids, may be used.

Citrus pulp from the first solvent extractor **25a** is fed to a desolventizer **30**. The desolventizer **30** removes solvent and water from the solids remaining after extraction, enabling the solvent to be reclaimed for future use and also ensuring that the product is safe for milling and commercial use. The desolventizer **30** can employ indirect heat to remove significant amounts of solvent from the solid residue. Alternatively, direct heat can be provided for drying, e.g., by providing hot air from flash dryers or fluidized bed dryers. Direct steam may be employed, if desired, to remove any trace amounts of solvent remaining in the solids. Vapors from the desolventizer **30** preferably are recovered and fed to the still **24** to reclaim at least a portion of the solvent.

Retention time in each extraction step may vary over a wide range but can be about 5 minutes or less per extraction step. The temperature in the solvent extractor(s) depends on such factors as the type of solvent used but most often ranges from about 4°C to about 85°C at atmospheric pressure. Temperatures can be appropriately increased or decreased for operation under super- or sub-atmospheric pressures. Optionally, techniques such as ultrasound are used for enhancing efficiency of the extraction process. By maintaining a closed system, solvent losses during extraction, desolventizing, and distillation can be minimized. Preferably, at least about 70 wt% of the solvent is recovered and reused. A solvent make-up stream delivers fresh solvent into the solvent tank **26** to replenish any solvent that is not recovered.

In a preferred embodiment, the process according to the present invention further comprises a comminuting or pulverizing step prior to desolventizing and drying. Suitable methods include, but are not limited to, grinding, milling, crushing, high speed mixing, or impingement. Comminution or pulverization can be beneficial to disintegrate any clumps or aggregates that are left after the removal of liquid with the belt filter pressing step. This step furthermore facilitates the removal of solvent. While not wishing to be bound by theory, it is believed that comminution or pulverization further opens the fibers. As a result of this, the solvent is more uniformly distributed and easier to be removed in the subsequent desolventization and drying step. In yet another preferred embodiment, the comminuting or pulverizing step is used in combination with adding and dispersing water or a blend of water and an organic solvent (as described hereinbefore) to enhance desolventization and drying, and achieve the desired humidity in the finally obtained citrus fiber for a particular end use.

In another preferred embodiment, the process according to the present invention further comprises a comminuting or pulverizing step after drying. This post-drying comminuting or pulverizing step may be carried out to further reduce the particle size of the citrus fiber, to improve flowability, dispersability, and/or hydration properties.

In yet another preferred embodiment, the process according to the present invention further comprises the step of subjecting the citrus pulp to a processing aid. Preferably, the processing aid is selected from the group consisting of enzymes, acids, bases, hydrocolloids, vegetable fiber, bleaching agent, and combinations thereof. Preferably, the processing aid is mixed with the citrus pulp prior to homogenization.

In one aspect of the present invention, the processing aid may be used to tailor the properties of the finally obtained citrus fiber.

Preferred enzymes include, but are not limited thereto, pectinase, protease, cellulase, hemicellulase and mixtures thereof. When enzymes are used, they are to be used prior to any heat treatment that would inactivate them, and preferably also prior to homogenization. Inactivation by heat treatment is however desired once the desired effect is achieved.

Preferred acids include, but are not limited thereto, citric acid, nitric acid, oxalic acid, ethylenediaminetetraacetic acid and combinations thereof. Citric acid is however most preferred as it is a food grade acid.

A preferred base is caustic soda.

Preferred hydrocolloids include, but are not limited thereto, pectin, alginate, locust bean gum, xanthan gum, guar gum, carboxymethylcellulose and combinations thereof.

A bleaching agent may further enhance the color properties (i.e. render the citrus fiber even more whiter). A preferred bleaching agent is hydrogen peroxide.

The citrus fiber obtained by the process according to the present invention has improved properties over other citrus fibers from the prior art. Especially, the citrus fiber has good swelling behavior, hydration ability and viscosifying properties. It is capable of building viscosity under relatively low shear.

The citrus fiber of the present invention has a c* close packing concentration of less than 3.8 w%, anhydrous basis. Preferably, the c* close packing concentration is less than 3.6, even more preferably less than 3.4, and most preferably less than 3.2 w%, anhydrous basis. The determination of the c* close packing concentration is described in the test method section herein below.

The citrus fiber preferably has a moisture content of 5% to 15%, more preferably from 6% to 14%. Preferably, at least 90% of the volume of the particles have a diameter of less than 1000 micrometers, preferably from 50 micrometers to 1000 micrometers, more preferably from 50 micrometers to 500 micrometers, even more preferably from 50 micrometers to 250 micrometers.

The citrus fiber preferably has a viscosity of at least 1000 mPa.s, wherein said citrus fiber is dispersed in standardized water at a mixing speed of from 800 rpm to 1000 rpm, preferably 900 rpm, to a 3 w/w% citrus fiber/standardized water solution, and wherein said viscosity is measured at a shear rate of $5\ s^{-1}$ at 20°C. Preferably, the viscosity at a shear rate of $5\ s^{-1}$ at 20°C is at least 2000 mPa.s, more preferably at least 3000 mPa.s, even more preferably at least 4000 mPa.s, even more preferably at least 5000 mPa.s and up to 15000 mPa.s. The preparation of the standardized water, and the method for measuring viscosity is described in the test method section herein below.

The citrus fiber according to the present invention further has good emulsification properties, as shown in the examples. The D4,3 value in the oil-rich phase is typically below 15 micrometers for the citrus fiber of the present invention.

In a preferred embodiment, the citrus fiber of the present invention also has excellent whiteness properties, even without the need for using bleaching agents. The citrus fiber typically has a CIELAB L* value of at least 85. But with the process according to the present invention, it is possible to obtain much higher L* values. Thus, according to another aspect, the present invention is directed to a citrus fiber having a CIELAB L* of at least 90, preferably at least 92, even more preferably at least 93. Preferably, the citrus fiber has a CIELAB b* value of less than 20, even more preferably of less than 15. The method for determining the CIELAB L* and b* values is described in the test method section herein below. As discussed hereinbefore, bleaching agents may still be used as processing aids in the process to even further improve the whiteness of the citrus fiber.

The citrus fiber according to the present invention can be blended with other fibers, such as plant-derived (e.g. from vegetables, grains/cereals) fibers, with other citrus fibers such as citrus fiber obtained from citrus peel or citrus rag, or combinations thereof. The blend can be in dry or liquid form.

In another aspect, the citrus fiber of the present invention and the blends described hereinbefore may be used in food applications, feed applications, beverages, personal care products, pharmaceutical products or detergent products. The amount of citrus fiber (or blend) to be used depends on the given application and the desired benefit to be obtained, and lies within the knowledge of a skilled person.

Food applications may include, but are not limited to, dairy products, frozen products, bakery products, fats and oils, fruit products, confectionary, meat products, soups, sauces and dressings. Dairy products include, but are not limited to yoghurt, fromage frais, quark, processed cheese, dairy desserts, mousses. Frozen products include, but are not limited to, ice cream, sorbet, water ice. Bakery products include, but are not limited to, cakes, sweet goods, pastry, patisserie, extruded snacks, fried snacks. Fats and oils include, but are not limited to, margarines, low fat spreads, cooking fats. Fruit products include, but are not limited to, fruit preparations, yoghurt fruit preparations, conserves, jams, jellies. Confectionary includes, but is not limited to, candy, chocolate spreads, nut-based spreads. Meat products include, but are not limited to, chilled or frozen processed meat and poultry, preserved meat products, fresh sausage, cured sausage and salami.

Beverages may include concentrates, gels, energy drinks, carbonated beverages, non-carbonated beverages, syrups. The beverage can be any medical syrup or any drinkable solution including iced tea, and fruit juices, vegetable based juices, lemonades, cordials, nut based drinks, cocoa based drinks, dairy products such as milk, whey, yogurts, buttermilk and drinks based on them. Beverage concentrate refers to a concentrate that is in liquid form.

Personal care products may include cosmetic formulations, hair care products such as shampoos, conditioners, creams, styling gels, personal washing compositions, sun-creams and the like.

Detergent products may include hard surface cleaning products, fabric cleaning or conditioning products, and the like.

Test methods

1. Preparation of Standardised water

Dissolve 10.0g NaCl (e.g. Merck 1.06404.1000, CAS [7647-14-5]) and 1.55g $CaCl_2.2H_2O$ (e.g. Merck 1.02382.1000, CAS [10035-04-8]) in low conductivity water (e.g. milli-Q Ultrapure Millipore 18.2 MQcm), and make up to 1 liter to prepare standardized water stock. Take a 100ml aliquot of the standardized water stock and make up to 1 liter with low conductivity water.

2. Measuring c* close packing concentration

2.1 Principle

Citrus fiber samples (n ≥ 10) are wetted with ethylene glycol, dispersed in standardised tap water, and subjected to the MCR301 controlled shear stress (CSS) oscillatory test. The citrus fiber dispersions are measured by 0.25w/w% increments in the range of 1.75-5.00w/w%. The linear viscoelastic range (LVR) complex moduli G* is plotted against concentration. The close-packing concentration c* is determined via the two tangents crossover method on a linear scale.

2.2 Apparatus

- Anton Paar MCR301 rheometer with coaxial cylinder configuration (TEZ150P-CF Peltier at 20°C) with vane probe ST24-2D/2V/2V-3D, grooved measuring cup CC27/T200/SS/P and circulating cooling water bath set at 15 °C. The equipment must be clean and dry, and the MCR301 units must be turned on 30 minutes before use. Checks should be made according to the instruction manual of the supplier (see instruction manual). The Circulator bath and pump should be at all times in use to avoid burning of the peltier unit. According to the manufacturer, the water bath must be filled with demineralised water containing maximum 30 % of antifreeze (e.g. ethylene glycol).

- RWD 20 Digital IKA stirrer and lower the paddle (4 bladed propeller 07 410 00)

- 600 ml Duran glass beaker (ø 10cm)

- Laboratory balance having a precision of 0.01 g

- Hard plastic soup spoon

2.3 Procedure

System Start-up

Start up the circulator bath (filled with demineralised water + 30% ethylene glycol (e.g. Merck 1.00949.1000, CAS [107-21-1])) and afterwards the rheometer according to the procedure explained in the instruction manual. Select the workbook and perform the initialisation procedure according to the instruction manual.

System calibration

The standard calibration check procedure for the MCR301 is fully described in the instruction manual and should be performed according to the instruction manual. The MCR301 instruments must be ready (initiated and all parameters checked) before testing the citrus fiber dispersions. The ST24 measuring system CSR should be set to 1 and the CSS value (Pa/mNm) should be fixed with certified calibration Newtonian oil (e.g. Cannon N100, available from Cannon Instrument Company, State College, PA 16803, USA).

Sample preparation

- Place a 600 ml glass beaker on the laboratory balance, and zero the balance.

- Weigh into the beaker the required grams (x) of citrus fiber, to the nearest 0.01 g, according to the moisture content (m) of the citrus fiber sample: x=3c/[(100-m)/100], for any given concentration c in w/w% (samples starting at 1.75 w/w%, to 5.00 w/w% with 0.25w/w% increments). The moisture content m should be determined by infra-red moisture balance (Sartorius MA 30), as weight loss at 105°C with automatic timing, typically 3-4g citrus fiber covering the entire bottom of the aluminium pan. The moisture content (m) of citrus fiber is in weight percent (w%).
- Weigh into a second 600mL beaker the required grams (w) of standardised tap water, to the nearest 0.1 g, according to the moisture of the citrus fiber sample: w=270.0-x
- Place the beaker with CPF on the laboratory balance, zero the balance, add 30.0g (to the nearest 0.1 g) of ethylene glycol, put the beaker out of the balance and mix the content with the plastic spoon thereby wetting the whole powder (this operation is performed within 60 seconds).
- Pour at once the standardised tap water on to the wet citrus fiber and mix the content with the plastic spoon by repeated clockwise and anti-clockwise rotations (this operation is performed within 60 seconds).
- Position the glass beaker with its content (citrus fiber, ethylene glycol, standardised tap water) underneath a RWD 20 Digital IKA stirrer and lower the paddle (4 bladed propeller 07 410 00) into the paste until 2cm from the bottom of the glass beaker.
- Adjust the paddle speed (rpm) to 900 rpm and stir 10 minutes at 900 rpm.
- Cover the beaker with aluminium foil and allow 24 hours rest prior measurement
- Pour the required amount of CPF dispersion into the cylindrical cup of the rheometer and insert immediately the vane probe ST24 (starch cell probe) into the cylinder containing the CPF dispersion

Sample analysis

- Perform CSS oscillatory test with the MCR301 according to the manual instructions, with 2 segments:

segment 1: non recording, 10 minutes at 20°C (equilibration)
segment 2: recording, 1971 seconds at 20°C, 50 measuring points integration time 100 to 10 seconds log, torque 1 to 10,000μNm log, frequency 1Hz

Results

At low stress, where the G* (*versus* stress) is showing constant plateau values, average the G* results over the linear viscoelastic range. Using the software "LVE Range", the end of the linear viscoelastic region in the CSS experiments can be determined.

Plot the LVR G* *versus* concentration. The first tangent at low concentration (below c*) has a much lower slope than the second tangent at high concentration (above c*). Using linear fitting (e.g. with Microsoft® Excel®), the crossover point of both tangents occurs at the close packing concentration c*.

3. Measuring Viscosity

Add citrus fiber to standardized water in a beaker with a paddle mixer to obtain a 3 wt% citrus fiber dispersion with a total volume of 300 ml. Prior to adding the citrus fiber, create a vortex by adjusting the paddle speed to 900 rpm using an IKA Overhead Mechanical Stirrer RW20 equipped with a 4-bladed propeller stirrer. Then add the citrus fiber quickly (before the viscosity builds up) on the walls of the vortex under stirring (900 rpm using an IKA Overhead Mechanical Stirrer RW20 equipped with a 4-bladed propeller stirrer). Continue stirring for 15 minutes at 900 rpm. Store the sample for 12 hours at 20°C.

Then perform the viscosity test with a rheometer (e.g. Anton Paar MCR300), in accordance with the rheometer's instructions, in function of shear rate (from 0.01 to 100 s$^{-1}$) at 20°C.

The viscosity (mPa.s) is determined at a shear rate of 5 s$^{-1}$.

4. Emulsification

Prepare an emulsion containing 20 wt% sunflower oil, 2wt% citrus pulp fiber and the remaining standardized tap water. First disperse the fiber in the water phase under high-shear mixing (8000 rpm) for 1 minute. Then add the oil to the water phase under high-shear mixing (13500rpm) for 5 min at room temperature and constant mixing speed.

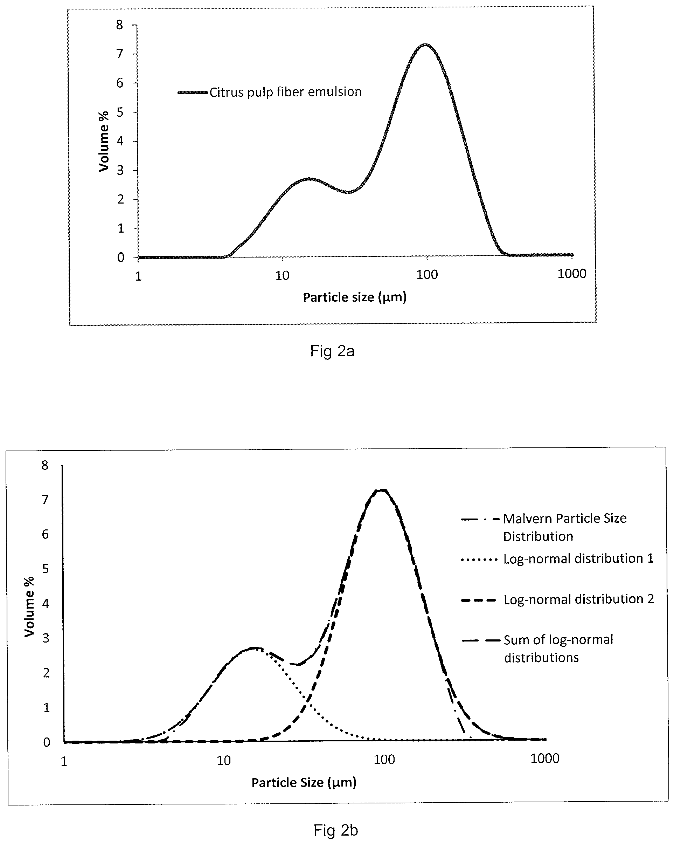

Particle size distribution of the obtained emulsions is measured using laser light scattering (e.g. using a Malvern MasterSizer X). Typically, a bimodal particle size distribution is observed (see Fig. 2a). The peak on the right corresponds with the particle size distribution of the oil-rich fraction of the emulsion (oil droplets + soluble fibers), while the peak on the left corresponds with the particle size distribution of the insolubles-rich fraction of the emulsion (e.g. cellulose).

The Malvern software allows the determination of an overall volume mean diameter D(4,3), but cannot provide the D(4,3) of the separate fractions. However, as fractions show a log-normal distribution, a peak deconvolution can be applied.

Peak deconvolution can be performed as follows: transfer the raw data from the Malvern MasterSizer X into Microsoft Excel™ for further analysis. It is assumed that the overall volume mean diameter (as obtained by the Malvern MicroSizer) equals the sum of 2 log-normal distributions.

The equation for a log-normal distribution can be found in literature. The lognormal distribution is a two-parameter distribution with parameters $\mu'$ and $\sigma_{T'}$. The probability density function for this distribution is given by:

$$f(T') = \frac{1}{\sigma_{T'}\sqrt{2\pi}}e^{-\frac{1}{2}\left(\frac{T'-\mu'}{\sigma_{T'}}\right)^2}$$

where $T' = \ln(T)$, where the $T$ values correspond with the particle sizes in the present method, and

$\mu'$ = mean of the distribution

$\sigma_{T'}$ = standard deviation of the distribution

Deconvolution can be performed based on this equation and the results obtained are shown in Fig 2b.

A good fit is found between the raw data distribution and the applied model. The mean ($\mu'$) of the peaks of each distribution corresponds with the D(4,3) of each phase (oil-rich phase and insolubles-rich phase). This assumption can be made due to the fact that the particles follow an almost perfect log-normal distribution.

5. Measuring Colour (CIELAB L*, b* values)

CIE *L*a*b* (CIELAB) is the most complete color space specified by the International Commission on Illumination (*Commission Internationale d'Eclairage*). It describes all the colors visible to the human eye and was created to serve as a device independent model to be used as a reference. The L* and b* values of the citrus fiber are obtained by placing citrus fiber (in powder form) in the glass cell (fill the cell to about a half) of the colorimeter and analyse the sample in accordance with the user's instructions of the colorimeter. The colorimeter used is a Minolta CR400 Colorimeter.

Examples

1. Examples 1-5

Orange pulp is adjusted with water to a dry matter content of 5 wt% to obtain 720 kg of the orange pulp. The pulp is charged to a pressure homogenizer (Niro Soavi, type NS3006L) and recirculated (maximum 5 bar) while adjusting the feed pressure to 700 bar.

The precipitation tank is filled with a centrifuge pump with 1.8 m$^3$ of 75-80 wt% ethanol solution from the first washing tank. The homogenized pulp is sent straight to the precipitation tank with a volumetric pump. Agitate while filling the tank, and continue stirring for about 30 minutes.

Adjust the speed of the centrifuge decanter (Flottweg centrifuge, 900R150, decanter Z23-3) to 5260 rpm. The differential speed is adjusted to 30 rpm and the diameter adjustment to 145mm. Charge the product to the centrifuge decanter with a volumetric pump, and recover the product.

First ethanol washing: a tank is filled with 1.5 m$^3$ of 82 wt% ethanol solution from the second ethanol washing. Feed the recovered product into the tank, and agitate for about 30 minutes. The washed product is then sent to a 100 $\mu$m rotative filter with a volumetric pump, and product is recovered.

Second ethanol washing: send the recovered product from the first ethanol washing to a tank filled with 1.4 m$^3$ of 85 wt% ethanol solution, and agitate for about 30 minutes. The washed product is then sent to a 100 $\mu$m rotative filter with a volumetric pump, and product is recovered.

The recovered product from the second ethanol washing is then fed to a screw press. The speed and pressure is adjusted to obtain a dry matter content of about 30 wt%.

The product is then milled using a Lödigue, 900M340, type FM300DIZ, and mill for 15 to 30 minutes.

The product is then fed to a vacuum dryer (ECI) and mixed for about 90 minutes. Add slowly 40% (based on dry matter content of the product) of a 60% ethanol solution. Dry with 95°C water for 4 hours under vacuum.

Recover the orange pulp fiber.

5 samples (Ex. 1-5) are prepared, originating from Brazilian orange pulp from different sources.

2. Comparative example

The orange pulp fiber obtained by the process of the present invention is compared to commercially available citrus fibers:

Citri-Fi 100 and Citri-Fi 100 M 40, orange fiber derived from orange pulp (Fiberstar Inc.)

Herbacel AQ-Plus Citrus Fibre F/100, and Herbacel AQ-Plus Citrus Fibre N, lemon fibers derived from lemon peel (Herbstreith & Fox Inc).

3. Results

3.1 c* close packing concentration

|  | c* (w%, anhydrous basis) |
|---|---|
| Example 4 | 2.80 |
| Example 5 | 3.09 |
| Citri-Fi 100 | 4.04 |
| Herbacel AQ-Plus Citrus Fibre N | 3.94 |

The c* close packing concentration of the citrus fiber according to the present invention is significantly lower than those of the commercially available fibers.

3.2 Viscosity

|  | viscosity (mPas) |
|---|---|
| Example 1 | 7085 |
| Example 2 | 4455 |
| Example 3 | 13000 |
| Example 4 | 6900 |
| Example 5 | 22890 |
| Citri-Fi 100 | 508 |
| Herbacel AQ-Plus Citrus Fibre F/100 | 250 |

A significant difference in viscosity is observed between the orange fibers according to the present invention (ex. 1-5), and the commercially available fibers.

Furthermore, an additional test has been carried out. It has been assessed at with mixing speed (versus the 900 rpm used in the test method), about the same level of viscosity increase could be obtained for a commercial citrus fiber. For the Citri-Fi 100 sample, a viscosity of 7545 mPa.s could be obtained if the citrus fiber is dispersed in the standardized water only at high shear rates (9500 rpm). This shows the benefit of the citrus fiber of the present invention in that it can build viscosity even when dispersed in solution at low shear rates. This means that the citrus fiber of the present invention is much easier to process and provides economical advantages (equipment and energy) over the fibers of the prior art.

3.3 Emulsification

|  | D4,3 ($\mu$m) oil-rich phase | D4,3 ($\mu$m) insolubles-rich phase |
|---|---|---|
| Example 1 | 7.7 | 74.0 |
| Example 5 | 9.2 | 77.5 |
| Citri-Fi 100 M 40 | 31.1 | 59.8 |
| Herbacel AQ-Plus Citrus Fibre F/100 | 18.1 | 113.9 |

It can be observed that the D4,3 value in the oil-rich phase is much lower for the orange fiber of the present invention (example 1, 5). This means that the oil droplets in this phase are much smaller and hence prove the better emulsification behavior of the orange fiber of the present invention.

3.4 Colour

|  | L* | b* |
|---|---|---|
| Example 1 | 93 | 12 |
| Citri-Fi 100 M 40 | 87 | 15.3 |
| Herbacel AQ-Plus Citrus Fibre F/100 | 88 | 11.7 |

The orange fiber of the present invention has an L* value above 90, and is whiter than the commercial fibers.

**Claims**

1. A process for preparing citrus fibers from citrus pulp, the process comprising

    a. treating citrus pulp using pressure homogenization to obtain homogenized citrus pulp;
    b. washing the homogenized citrus pulp with an organic solvent to obtain organic solvent washed citrus pulp;
    c. desolventizing and drying the organic solvent washed citrus pulp; and
    d. recovering citrus fiber therefrom.

2. A process according to claim 1, wherein the treatment comprises pressure homogenization using a pressure of from 50 bar to 1000 bar.

3. A process according to claim 2, wherein said treatment is a single-pass pressure homogenization using a pressure of from 300 bar to 1000 bar.

4. A process according to claim 2, wherein said treatment is a multi-pass pressure homogenization comprising at least 2 passes, using a pressure of from 100 bar to 600 bar.

5. A process according to any of the preceding claims, wherein the citrus pulp is subjected to a heat treatment prior to the homogenization treatment at a temperature of from 50 °C to 140°C for a period of 1 second to 3 minutes.

6. A process according to any of the preceding claims, wherein said process further comprises a comminution or pulverizing step prior to desolventizing and drying.

7. A process according to any of the preceding claims, wherein said process further comprises a comminution or pulverizing step after drying.

8. A process according to any of the preceding claims, wherein said process further comprises subjecting said citrus pulp to a processing aid selected from the group consisting of enzymes, acids, bases, hydrocolloids, vegetable fiber, bleaching agents, and combinations thereof.

9. Citrus fiber obtained by the process of claims 1-8 having a c* close packing concentration value of less than 3.8 w%, anhydrous base.

10. Citrus fiber according to claim 9, wherein said citrus fiber has a moisture content of from 5% to 15%.

11. Citrus fiber according to claims 9-10 having a viscosity of at least 1000 mPa.s, wherein said citrus fiber is dispersed in standardized water at a mixing speed of from 800 rpm to 1000 rpm, to a 3 w/w% citrus fiber/standardized water solution, and wherein said viscosity is measured at a shear rate of 5 s$^{-1}$ at 20°C.

12. Citrus fiber according to claims 9-11 having a CIELAB L* value of at least 90.

13. Citrus fiber according to claim 12 having a CIELAB b* value of less than 20.

**Patentansprüche**

1. Verfahren zur Herstellung von Zitrusfaser aus Zitrusfruchtfleisch, das Verfahren umfassend

a. Behandlung von Zitrusfruchtfleisch unter Verwendung von Druckhomogenisierung, um homogenisiertes Zitrusfruchtfleisch zu gewinnen;
b. Waschen des homogenisierten Zitrusfruchtfleischs mit einem organischen Lösungsmittel, um mit organischem Lösungsmittel gewaschenes Zitrusfruchtfleisch zu gewinnen;
c. Desolventierung und Trocknung des mit organischen Lösungsmitteln gewaschenen Zitrusfruchtfleischs; und
d. Gewinnung von Zitrusfaser daraus.

2. Verfahren nach Anspruch 1, wobei die Behandlung eine Druckhomogenisierung unter Verwendung eines Drucks von 50 bar bis 1000 bar umfasst.

3. Verfahren nach Anspruch 2, wobei die Behandlung eine Druckhomogenisierung mit einem einzigen Durchgang ist, wobei ein Druck von 300 bar bis 1000 bar verwendet wird.

4. Verfahren nach Anspruch 2, wobei die Behandlung eine Druckhomogenisierung mit mehreren Durchgängen ist, die mindestens 2 Durchgänge umfasst, wobei ein Druck von 100 bar bis 600 bar verwendet wird.

5. Verfahren nach einem der vorstehenden Ansprüche, wobei das Zitrusfruchtfleisch vor der Homogenisierungsbehandlung einer Wärmebehandlung bei einer Temperatur von 50 °C bis 140 °C für eine Dauer von 1 Sekunde bis 3 Minuten unterzogen wird.

6. Verfahren nach einem der vorstehenden Ansprüche, wobei das Verfahren weiter einen Zerkleinerungs- oder Pulverisierungsschritt vor der Desolventierung und Trocknung umfasst.

7. Verfahren nach einem der vorstehenden Ansprüche, wobei das Verfahren weiter einen Zerkleinerungs- oder Pulverisierungsschritt nach der Trocknung umfasst.

8. Verfahren nach einem der vorstehenden Ansprüche, wobei das Verfahren weiter das Unterziehen des Zitrusfruchtfleischs einem Verarbeitungshilfsmittel, ausgewählt aus der Gruppe bestehend aus Enzymen, Säuren, Basen, Hydrokolloiden, Pflanzenfasern, Bleichmitteln und Kombinationen davon, umfasst.

9. Nach dem Verfahren der Ansprüche 1-8 gewonnene Zitrusfaser, aufweisend einen Packungsdichte-Konzentrationswert c* von weniger als 3,8 Gew.-%, wasserfreie Basis.

10. Zitrusfaser nach Anspruch 9, wobei die Zitrusfaser einen Feuchtigkeitsgehalt von 5% bis 15% aufweist.

11. Zitrusfaser nach den Ansprüchen 9-10, aufweisend eine Viskosität von mindestens 1000 mPa.s, wobei die Zitrusfaser in standardisiertem Wasser bei einer Mischgeschwindigkeit von 800 U/min bis 1000 U/min zu einer Lösung aus Zitrusfaser/standardisiertem Wasser von 3 Gew./Gew.-% dispergiert wird, und wobei die Viskosität bei einer Scherrate von 5 s$^{-1}$ bei 20 °C gemessen wird.

**12.** Zitrusfaser nach den Ansprüchen 9-11, aufweisend einen CIELAB L\*-Wert von mindestens 90.

**13.** Zitrusfaser nach Anspruch 12, aufweisend einen CIELAB b\*-Wert von weniger als 20.

**Revendications**

**1.** Procédé de préparation de fibre d'agrume à partir de pulpe d'agrume, le procédé comprenant

a. le traitement de la pulpe d'agrume par homogénéisation sous pression pour obtenir une pulpe d'agrume homogénéisée ;
b. le lavage de la pulpe d'agrume homogénéisée avec un solvant organique pour obtenir une pulpe d'agrume lavée au solvant organique ;
c. la désolvantisation et le séchage de la pulpe d'agrume lavée au solvant organique ; et
d. la récupération de fibre d'agrume qui en résulte.

**2.** Procédé selon la revendication 1, dans lequel le traitement comprend une homogénéisation sous pression en utilisant une pression de 50 bar à 1 000 bar.

**3.** Procédé selon la revendication 2, dans lequel ledit traitement est une homogénéisation sous pression en une passe en utilisant une pression de 300 bar à 1 000 bar.

**4.** Procédé selon la revendication 2, dans lequel ledit traitement est une homogénéisation de pression à passes multiples comprenant au moins 2 passes, en utilisant une pression de 100 bar à 600 bar.

**5.** Procédé selon l'une quelconque des revendications précédentes, dans lequel la pulpe d'agrume est soumise à un traitement thermique avant le traitement d'homogénéisation à une température de 50 °C à 140 °C pendant une période de 1 seconde à 3 minutes.

**6.** Procédé selon l'une quelconque des revendications précédentes, dans lequel ledit procédé comprend en outre une étape de broyage ou de pulvérisation avant la désolvantisation et le séchage.

**7.** Procédé selon l'une quelconque des revendications précédentes, dans lequel ledit procédé comprend en outre une étape de broyage ou de pulvérisation après le séchage.

**8.** Procédé selon l'une quelconque des revendications précédentes, dans lequel ledit procédé comprend en outre la soumission de ladite pulpe d'agrume à un auxiliaire de traitement sélectionné à partir du groupe constitué des enzymes, des acides, des bases, des hydrocolloïdes, des fibres végétales, des agents blanchissants et leurs combinaisons.

**9.** Fibre d'agrume obtenue par le procédé des revendications 1 à 8 ayant une valeur de concentration après compactage c\* inférieure à 3,8 % en poids, base anhydre.

**10.** Fibre d'agrume selon la revendication 9, dans laquelle ladite fibre d'agrume a une teneur en humidité de 5 % à 15 %.

**11.** Fibre d'agrume selon les revendications 9 à 10, ayant une viscosité d'au moins 1 000 mPa.s, dans laquelle ladite fibre d'agrume est dispersée dans de l'eau normalisée à une vitesse de mélange de 800 tr/min à 1 000 tr/min, en une solution de 3 % m/m de fibre d'agrume/eau normalisée, et dans laquelle ladite viscosité est mesurée à un taux de cisaillement de 5 s$^{-1}$ à 20 °C.

**12.** Fibre d'agrume selon les revendications 9 à 11 ayant une valeur CIELAB L\* d'au moins 90.

**13.** Fibre d'agrume selon la revendication 12, ayant une valeur CIELAB b\* inférieure à 20.

Fig. 1

Fig 2a

Fig 2b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7094317 B
- WO 9427451 A

- WO 2006033697 A